**Europäisches Patentamt**

**European Patent Office**

**Office européen des brevets**

(11) Numéro de publication: **0 086 315**
A1

(12) **DEMANDE DE BREVET EUROPEEN**

(21) Numéro de dépôt: **82402127.3**

(22) Date de dépôt: **23.11.82**

(51) Int. Cl.³: **B 01 D 11/02, A 61 K 35/78**

(30) Priorité: **22.12.81 FR 8123908**

(43) Date de publication de la demande: **24.08.83**
**Bulletin 83/34**

(84) Etats contractants désignés: **AT BE CH DE FR GB IT LI LU NL SE**

(71) Demandeur: **PRODIPHARM S.A., 1, Grand' Places, CH-1701 Fribourg (CH)**

(72) Inventeur: **De Boccard, Hubert, 1 Grand Places, Fribourg (CH)**

(74) Mandataire: **Burtin, Jean-François, 5 Bis, rue Parmentier, F-92200 Neuilly S/Seine (FR)**

(54) Nouveau procédé d'obtention de substances vaso-actives extraites de feuilles de ginkgo.

(57) La présente invention a pour objet un nouveau procédé d'obtention des substances vaso-actives des feuilles ancore vertes de Gingko Biloba par épuisement préalable avec un solvant hydrocarboné, extraction par une cétone aqueuse puis purification sélective de l'extrait recueilli par passage sur un polymère de vynil pyrrolidone.

L'extrait de Gingko Biloba trouve un emploi en thérapeutique sous forme de compositions pharmaceutiques.

EP 0 086 315 A1

Nouveau procédé d'obtention de substances vaso-actives
extraites des feuilles de GINKGO.

La présente invention a pour objet un nouveau procédé
d'obtention des principes actifs du Ginkgo biloba.

Elle a plus particulièrement pour objet un nouveau procédé
de préparation par épuisement des feuilles vertes de Gingko
Biloba et purification selective de l'extrait recueilli.

Les feuilles de Gingko Biloba renferment un grand nombre de
constituants dont certains sont actifs sur la circulation,
comme par exemple des Ginkgolides et des flavonoïdes,
(The Chemistry of Natural Products Vol 4 (1907) 89) et
d'autres sont seulement un ballast inactif. Parmi les
flavonoïdes du Ginkgo Biloba on a determiné la présence de
gallocatechine, de galloepicatichine, de pro-delphinidines,
de glycosides de flavones et de composés bis-flavoniques.

Tous ces composés ne sont pas intéressants du point de vue
thérapeutique et l'objectif du procédé d'extraction est d'être
le plus selectif possible. On cherche, en particulier, à
éliminer des substances à caractère phénolique, qui constituent
une charge inutile, et des substances lipophiles. Ces
dernières substances nuisent à la stabilité des compositions
pharmaceutiques injectables.

C'est pourquoi le procédé décrit dans le brevet allemand
1.767.098 comportant une phase d'épuisement avec une cétone
aqueuse puis une phase d'extraction de l'extrait cétonique par
un solvant chloré, permet d'obtenir un totum qui convient
seulement à la fabrication de formes pharmaceutiques ingérables
mais qui ne convient pas pour la fabrication de formes
pharmaceutiques injectables.

Le brevet français 2.132.761 décrit un perfectionnement à ce procédé qui consiste à traiter le totum de principes actifs extraits par le sulfate d'ammonium, à procéder à un nouvel épuisement par la methylethylcétone, à séparer la solution cétonique que l'on amène à sec, à reprendre le résidu par une solution alcoolique, à soumettre cette solution alcoolique par un sel insoluble de plomb, à filtrer le précipité, à traiter à nouveau le filtrat par du sulfate d'ammonium, à extraire à nouveau la methylethylcétone à concentrer cette solution organique et à la traiter avec un polyamide pulvérulent à haut poids moléculaire (Nylon 6). On sépare ainsi par adsorption des substances gênantes. Le filtrat amené à sec est repris par l'éthanol. Les principes actifs précipitent au repos.

On obtient ainsi après une cascade d'extractions, d'évaporation puis de reprises par des solvants 1200 g de substances actives au départ de 100 kg de feuilles de Gingko Biloba grossièrement broyées. Ce procédé se caractèrise, donc, par la multiplicité des étapes et par conséquent par un rendement faible en principe actif. En outre, la précipitation par le plomb est peu selective. Elle précipite des composés autres que les phénols, sans pour autant éliminer tous les produits phénoliques.

La présente invention concerne donc un procédé amélioré qui comporte moins d'étapes, qui assure une purification plus facile et plus sélective et enfin qui assure un meilleur rendement en principes actifs.

Ce nouveau procédé se caractèrise en ce que l'on soumet les feuilles encore vertes de Gingko Biloba à un épuisement par un solvant hydrocarboné permettant d'éliminer d'emblée des substances lipophiles, recueille l'insoluble que l'on extrait avec un solvant cetonique aqueux, sépare la phase cetonique, l'additionne de sulfate d'ammonium et d'une cetone aliphatique insoluble dans l'eau, concentre la phase cetonique

./...

puis la dilue avec un alcanol aqueux, l'acidifie par addition d'un acide minéral ou organique aqueux traite la solution hydroorganique par addition d'un polymère insoluble de vinyl pyrrolidone préalablement purifié, sépare l'insoluble par filtration ou centrifugation puis neutralise le filtrat ou le surnageant que l'on amène à siccité.

On obtient ainsi un rendement final plus élevé en principes actifs plus purs et utilisables pour toutes les formes d'administration pharmaceutique

Ce nouveau procédé se caractèrise encore par les modes d'exécution suivants actuellement préférés :

a) le solvant d'extraction hydrocarboné est un hydrocarbure liquide ou un mélange liquide d'hydrocarbures à poids moléculaire élevé comme l'ether de pétrole, la ligroïne ou le Kérosène.

b) le solvant cétonique aqueux est l'acétone diluée avec une quantité d'eau comprise entre 20 et 60 %.

c) la cétone aliphatique insoluble est la methylethylcetone, la diethylcetone ou la methylisobutylcetone.

d) l'acidification est contrôllée de telle sorte que le pH soit compris entre 3 et 4 et de préférence soit de 3,5.

e) l'alcanol aqueux est un mélange d'un alcanol de bas poids moléculaire, comme le méthanol, l'éthanol ou le propanol, et d'eau.

f) le polymère insoluble de vinylpyrolidone est une polyvinylpyrolidone de poids moléculaire moyen de 70.000 comme celle vendue sous la marque KOLLIDON CE 5145 par la firme BASF ou une polyvinylpolypyrolidone pontée comme celle vendue sous la marque POLYCLAR AT par la firme General Aniline and Film Corp.

g) le polymère insoluble de vinylpyrrolidone est purifié au préalable par traitement par le ferricyanure de potassium en milieu alcalin.

./...

h) le filtrat ou le surnageant de centrifugation est neutralisé par addition très ménagée de soude ou de carbonate de sodium.

i) Le filtrat amené à sec est repris par l'éthanol, filtré et amené à sec à nouveau pour fournir le mélange de principes actifs.

L'extrait ainsi obtenu manifeste des propriétés vasodilatatrices et spasmolytiques prononcées et trouve de ce fait un emploi en thérapeutique. Il est employé sous forme de compositions pharmaceutiques notamment pour la voie buccale telles que comprimés nus ou enrobés, gouttes, solutions ou suspensions buvables en ampoules ou en flacons.

L'exemple suivant illustre l'invention. Il ne la limite en aucune façon.

10 kg de feuilles encore vertes (les feuilles jaunes sont plus riches en biflavonoïdes) de GINKGO BILOBA sont réduites en poudre grossière et apuisées par 150 l d'éther de pétrole 40-65 à l'aide d'un appareil de Soxhlet, pendant 48 heures en continu. Les lipides et les pigments (chlorophylle et xanthophylle) sont aussi éliminés. Le solvant est ensuite récupéré par distillation.

Le marc, étalé sur une surface plane, est séché à l'air, puis extrait par 40 litres d'acétone aqueuse 70 : 30, à reflux. La température est de l'ordre de 55 °C. Après huit heures, l'extrait est filtré et la marc pressé fortement.

La solution hydro-acétonique est additionnée de 3 kg de sulfate d'ammonium, et de 3 l de Méthylethylcetone. La phase aqueuse ainsi relarguée est soutirée et la phase cétonique recueillie est séchée complètement par addition de 2 kg de sulfate d'ammonium. Après contact d'une heure, les cristaux sont séparés par filtration sous vide, lavés par 3 l d'acétone. Le filtrat et le solvant de lavage, réunis, sont concentrés à l'évaporateur rotatif sous vide jusqu'à l'obtention d'un concentrat de 0.5 vol.

Ce résidu est additionné d'alcool à 50° pour avoir une concentration 1,5 vol.

La solution ainsi obtenue, contient 10 % de résidu sec.

A ce stade, il est nécessaire de séparer la fraction phénolique.

La solution alcoolo-acétonique à environ 10 % est diluée au 1/10 et amenée à pH = 3.5 par addition d'une solution aqueuse N d'acide acétique. On ajoute alors 0.1 p de poly vinyl pyrrolidone insoluble, purifiée comme suit. Ce traitement vise à la priver des impuretés réductrices et à lui conférer une granulométrie convenable.

Purification : lavage par une solution aqueuse contenant 20 % de $Na_2CO_3$, 9 % de NaCl, 0.25 % de ferricyanure de K. Le lavage se fait par quatre parties de solution pour une partie de poly vinyl pyrrolidone, sous agitation jusqu'à décoloration de la solution. Après filtration sous vide, le lavage est repris par une nouvelle quantité de solution de purification. Ces lavages successifs sont poursuivis jusqu'à ce qu'il n'y ait plus décoloration de la solution après une agitation d'au moins trois heures. Enfin, la poly vinyl pyrrolidone est lavée successivement avec à chaque fois une agitation d'une heure, par l'eau distillée, une solution de $Na_2CO_3$ à 10 %, une solution de HCI à 10 %, de l'eau distillée et de l'acétone.

Le polymère (Kollidon CE 5145 commercialisé par la firme BASF) est alors séché à l'air à 60°C, tamisé sur un tamis de 40 Mesh et conservé à l'exsiccateur.

Après addition du polymère à l'extrait de GINKGO BILOBA, une agitation d'une demi-heure est maintenue. Puis le polymère est séparé par centrifugation à 3000 tours pendant 20 minutes, ou filtration sous vide.

Le filtrat (ou le surnageant) est neutralisé par une faible quantité d'un agent alcalin (Soude de carbonate mais ce dernier fournissant de la mousse, il est nécessaire d'ajouter quelques gouttes d'un agent anti-mousse) concentré sous vide à l'évaporateur rotatif jusqu'à siccité. Le résidu sec est dissout dans 41,5 vol. d'éthanol.

./...

Après repos de douze heures, cette solution est filtrée et le filtrat concentré sous pression réduitejusqu'à siccité.

On obtient un rendement final de 1.5 % environ.

REVENDICATIONS

1. Un nouveau procédé d'obtention des substances vaso-actives des feuilles de GINGKO BILOBA caractérisé en ce que l'on soumet les feuilles encore vertes de GINGKO BILOBA à un épuisement par un solvant hydrocarboné, recueille l'insoluble que l'on extrait par un solvant cétonique aqueux, sépare la phase organique que l'on additionne de sulfate d'ammonium et d'une cétone aliphatique insoluble dans l'eau, sépare la phase cétonique, concentre celle-ci puis la dilue avec un alcanol aqueux, l'acidifie par addition d'un acide minéral ou organique aqueux, traite cette solution acide par un polymère insoluble de Vinylpyrrolidone préalablement purifié, sépare le polymère par filtration ou centrifugation, neutralise le filtrat ou le supernageant et l'amène à sec.

2. Un mode d'exécution du procédé selon la revendication 1. dans lequel le solvant d'extraction hydrocarboné est un hydrocarbure liquide, ou un mélange d'hydrocarbure à poids moléculaire élevé.

3. Un mode d'exécution du procédé selon la revendication 1. dans lequel le solvant cétonique aqueux est l'acétone diluée avec une quantité d'eau comprise entre 20 et 60 %.

4. Un mode d'exécution du procédé selon la revendication 1. dans lequel l'acétone aliphatique insoluble est la méthylisobutylcétone, la diethylcétone ou la méthylisobutylcétone.

5. Un mode d'exécution du procédé selon la revendication 1. dans lequel l'acidification est contrôlée de telle sorte que le pH soit compris entre 3 et 4 et de préférence soit de 3,5.

6. Un mode d'exécution du procédé selon la revendication 1. dans lequel l'alcanol aqueux est un mélange d'un alcanol de bas poids moléculaire et d'eau.

7. Un mode d'exécution du procédé selon la revendication 1. dans lequel le polymère insoluble de vinylpyrolidone est une polyvinylpyrolidone de poids moléculaire poyen de 70.000 ou une polyvinylpolypyrrolidone.

0086315

8. Un mode d'exécution du procédé selon la revendication 1. dans lequel le polymère insoluble de vinylpyrrolidone est purifié au préalable par traitement par le ferricyanure de potassium en milieu alcalin.

Office européen des brevets

**RAPPORT DE RECHERCHE EUROPEENNE**

Numéro de la demande

EP 82 40 2127

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl. 3) |
|---|---|---|---|
| D,A | FR-B-2 132 761 (W. SCHWABE)<br>* Revendications 1, 2 * | 1 | B 01 D 11/02<br>A 61 K 35/78 |
| | --- | | |
| D,A | DE-B-1 767 098 (DR. W. SCHWABE)<br>* Revendications 1-3 * | 1 | |
| | --- | | |
| A | Chemical Abstracts, vol. 67, no 4, 24 juillet 1967, Columbus, Ohio, USA M. MARUYAMA et al. "The ginkgolides. I. Isolation and characterization of the various groups", page 2064, colonne 1, abstract no 21756z & Tetrahedron Lett., vol. 4, 1967, pages 299-302 | 1 | |
| | --- | | |
| A | Chemical Abstracts, vol 81, no 8, 11 novembre 197 4, Columbus, Ohio, USA P. ROVESTI "Cosmetological action of Gingko biloba total extract", page 416, colonne 1, abstract no 12 6679u & Riv. Ital. Essenze, Profumi, Piante Off., Aromi, Saponi, Cosmet., Aerosol, vol. 56, no 1, 1974, pages 13-17 | 1 | DOMAINES TECHNIQUES RECHERCHES (Int. Cl. 3)<br><br>B 01 D 11/00<br>A 61 K 35/78<br>C 07 G 17/00 |
| | ----- | | |

Le présent rapport de recherche a été établi pour toutes les revendications

| Lieu de la recherche<br>BERLIN | Date d'achèvement de la recherche<br>31-03-1983 | Examinateur<br>KUEHN P |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82